# EUROPEAN PATENT SPECIFICATION

(11) **EP 2 751 081 B1**
(45) Date of publication and mention of the grant of the patent: **04.01.2017**
(21) Application number: 12756750.1
(22) Date of filing: 12.09.2012
(51) Int. Cl.: C07D 215/14, B01D 9/02, A61K 31/47, A61P 3/00, A61P 9/00

(54) **POLYMORPHIC FORM OF PITAVASTATIN CALCIUM**
POLYMORPHE FORM VON PITAVASTATIN KALZIUM
FORME POLYMORPHE DE CALCIUM DE PITAVASTATINE

(30) Priority: 12.09.2011 SI 201100359 P
(43) Date of publication of application: 09.07.2014
(73) Proprietor: Farma GRS, d.o.o., 8000 Novo mesto (SI)
(72) Inventor: KLJAJIC, Alen, 3000 Celje (SI); TROST, Sabina, 1330 Kocevje (SI); PECAVAR, Anica, 8000 Novo mesto (SI); ZUPET, Rok, 1000 Ljubljana (SI)
(74) Representative: Uexküll & Stolberg
(86) International application number: PCT/EP2012/067859
(87) International publication number: WO 2013/037838

(56) References cited:
- WO-A1-2004/072040
- WO-A1-2005/063711
- WO-A1-2011/105649
- WO-A1-2012/063254
- WO-A2-2007/132482
- CN-A- 101 195 603
- KR-A- 20100 125 124
- Igor Ivanisevic ET AL: "Uses of X-Ray Powder Diffraction In the Pharmaceutical Industry", Pharmaceutical Sciences Encyclopedia: Drug Discovery, Development, and Manufacturing, 25 June 2010 (2010-06-25), pages 1-42, XP055007590, DOI: 10.1002/9780470571224.pse414 Retrieved from the Internet: URL:http://onlinelibrary.wiley.com/store/1 0.1002/9780470571224.pse414/asset/pse414.p df?v=1&t=gsssyq3r&s=316303db60793cc15e15c8 7a2d3b6ec61b51414e [retrieved on 2011-09-20]
- CAIRA: "Crystalline Polymorphism of Organic Compounds", TOPICS IN CURRENT CHEMISTRY, SPRINGER, BERLIN, DE, vol. 198, 1 January 1998 (1998-01-01), pages 163-208, XP008166276, ISSN: 0340-1022

## Description

### Field of the invention

The present invention relates to a new polymorphic form of pitavastatin calcium, a process for preparation thereof, to pharmaceutical formulations containing it and to its uses. The polymorphic form according to the present invention is characterized by high polymorphic purity and high chemical and physical stability.

### Background of the invention

Pitavastatin is a common name for (3R,5S,6E)-7-[2-cyclopropyl-4-(4-fluorophenyl)quinolin-3-yl]-3,5-dihydroxyhept-6-enoic acid. Pitavastatin can be represented by the following structural formula:

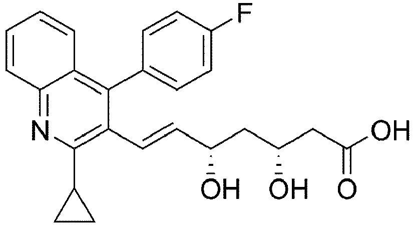

Pitavastatin is available on the market in form of pitavastatin calcium salt (2:1), hereinafter referred to as pitavastatin calcium. It is sold under the trademark Livazo, as a film coated tablet in 1 mg, 2mg, 4mg strengths.

Pitavastatin calcium is known by the chemical name monocalcium bis[(3R, 5S, 6E)-7-(2-cyclopropyl-4-(4-fluorophenyl)-3-quinolyl)-3,5-dihydroxy-6-heptenoate] and can be represented by the following structural formula:

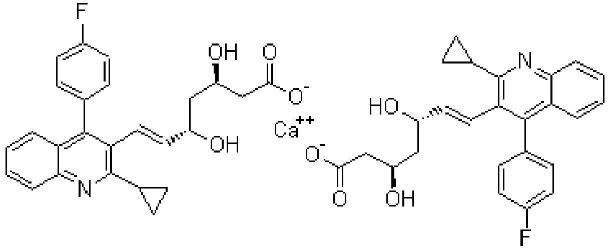

Pitavastatin is a potent statin that combines effective control of low density lipoprotein-cholesterol (LDL-C) and triglycerides (TG), with long-term incremental high density lipoprotein-cholesterol (HDL-C) increase and, due to its novel structure, is less likely to have drug-to-drug interactions.

It is indicated for reduction of elevated total cholesterol (TC) and LDL-C, in adult patients with primary hypercholesterolemia, including heterozygous familial hypercholesterolemia, and / or mixed dyslipidemia.

Several pitavastatin calcium polymorphic forms (Forms A, B, C, D, E, F) and amorphous form have been reported.

WO2004/072040 discloses polymorphic forms A, B, C, D, E, and F, and the amorphous form of pitavastatin calcium. It is reported that Form A can be generally prepared from pitavastatin sodium upon reaction with CaCl2 in an aqueous reaction medium. It may contain up to 15% water, preferably about 3 to 12%, more preferably 9 to 11% of water. Further it is reported that Forms B to F can be obtained by treating form A in the following ways:
- Form B can be prepared by suspending form A in ethanol containing water as a co solvent.
- Form C can be prepared by suspending form A in isopropanol containing water as a co solvent. Form C can also be prepared from a mixture of isopropanol and a ketone solvent, containing water as a co solvent.
- Form D can be prepared by suspending form A in absolute ethanol.
- Form E can be prepared by suspending form A in 1,4-dioxane containing water as a co solvent.
- Form F can be generally prepared by suspending form A in methanol containing water as a co solvent.

WO2005063711 discloses polymorphic form A of pitavastatin calcium which contains from 5 to 15% of water and which shows, in its X-ray powder diffraction as measured by using CuKa radiation, a peak having a relative intensity of more than 25% at a diffraction angle (20) of 30.16°. It is further reported that when pitavastatin calcium in polymorphic form A is subjected to drying in a usual manner the crystallinity decreases to a state close to amorphous state and the water content becomes to be at most 4%. It is shown that the amorphous form has very poor stability during the storage. The drawback of the form A disclosed in WO2005063711 is the extremely high sensibility to drying conditions, wherein the water content has to be carefully controlled by strict drying conditions. Any deviation in the process of drying may lead to formation of undesired forms, such as amorphous form.

Polymorphic forms of pitavastatin calcium are disclosed also in WO2011/105649, CN 101195603 and KR201000125124.

Polymorphic forms are of particular interest due to the provision of essentially pure compounds permitting an exact characterization of physico-chemical properties and possible favorable pharmacological characteristics, such as improved solubility, stability and particle size.

Consequently, there is still a need for an improved and reliable polymorphic form which would be reproducible on large scale, stable at standard technological processes such as drying, milling, granulation, and compression, stable on storing conditions and prepared by a process affording high yields, high purity and desired particle size distribution.

We have surprisingly found out that the new polymorphic form according to the present invention resists any conversion and is stable under technological process conditions such as drying, milling, compression, granulation or blending with excipients.

### Figures

Figure 1 shows an X-ray diffraction pattern (XRDP) of pitavastatin calcium in polymorphic form K
Figure 2 shows thermogravimetric (TG) curve of pitavastatin calcium in polymorphic form K
Figure 3 shows FT-IR spectrum of pitavastatin calcium in polymorphic form K
Figure 4 shows DSC curve of pitavastatin calcium in polymorphic form K

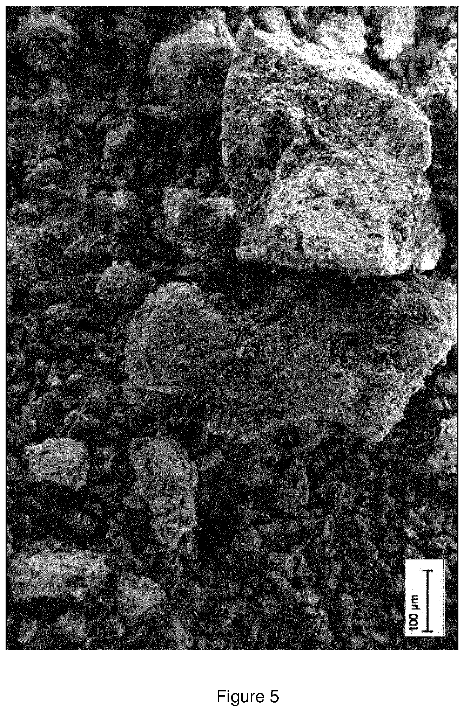

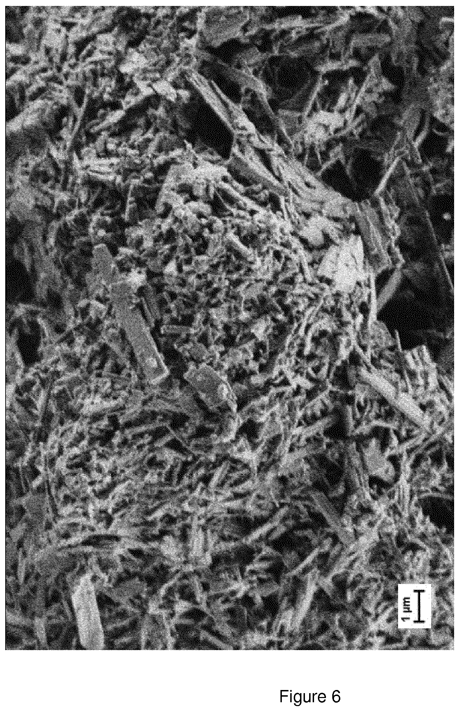

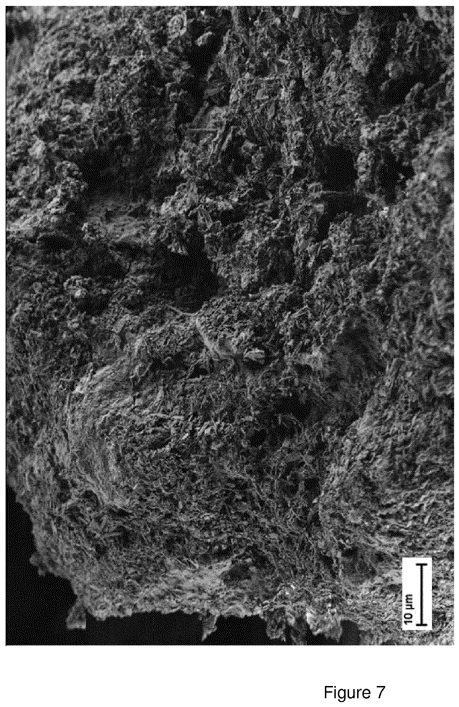

Figures 5, 6, 7 show a scanning electron microscope (SEM) image of pitavastatin calcium in polymorphic form K obtained in Example 1.
Figure 8 shows X-ray diffraction patterns (XRPD) of pitavastatin calcium in polymorphic form K during thermal treatment.
Figure 9 shows X-ray diffraction patterns (XRPD) of pitavastatin calcium in polymorphic form A during thermal treatment.

### Detailed description of the invention

A first aspect of the present invention provides pitavastatin calcium in polymorphic form K. Polymorphic form K of pitavastatin calcium is characterized by a powder X-ray diffraction pattern with the following peaks (° 2θ ± 0.2) and inter-planar spacing d [Å]:

| No. | Pos. [°2θ.] | d-spacing [Å] |
|---|---|---|
| 1 | 3,8 | 23,27 |
| 2 | 11,3 | 7,85 |
| 3 | 17,4 | 5,11 |
| 4 | 18,1 | 4,91 |
| 5 | 24,6 | 3,63 |

Preferably, the polymorphic form K is further characterized by a powder X-ray diffraction pattern with the following peaks (° 2θ ± 0.2) and inter-planar spacing d [A]:

| No. | Pos. [°2θ.] | d-spacing [Å] |
|---|---|---|
| 1 | 3,8 | 23,27 |
| 2 | 5,3 | 16,62 |
| 3 | 11,3 | 7,85 |
| 4 | 15,4 | 5,74 |
| 5 | 17,4 | 5,11 |
| 6 | 18,1 | 4,91 |
| 7 | 19,2 | 4,61 |
| 8 | 20,6 | 4,32 |
| 9 | 22,3 | 3,98 |
| 10 | 24,6 | 3,63 |

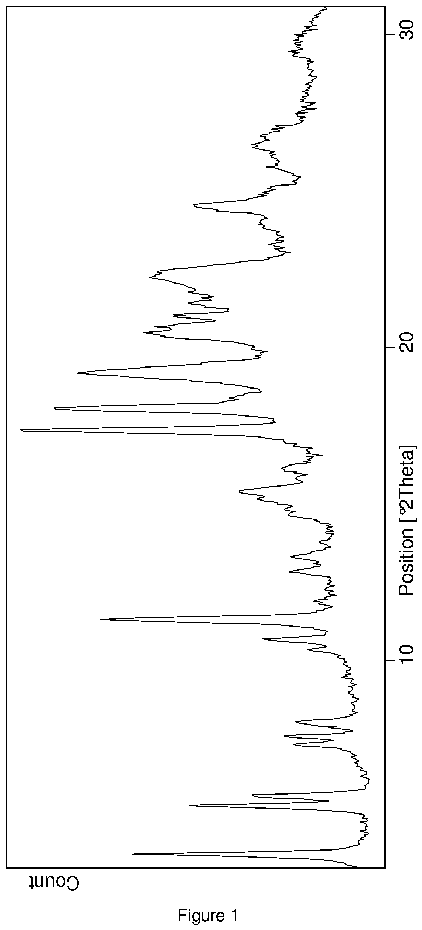

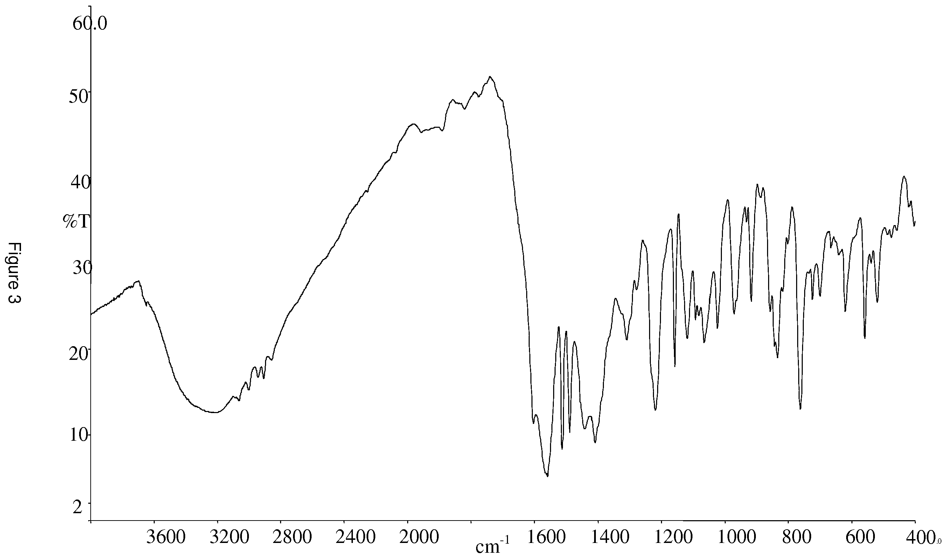

Polymorphic form K of pitavastatin calcium is further characterized by an XRPD spectrum as substantially shown in Figure 1 and in particular by an FT-IR as substantially shown in Figure 3.

Polymorphic form K of pitavastatin calcium exhibits an FT-IR spectrum with the following characteristic absorption bands at about 3210, 2944, 2909, 1820, 1603, 1559, 1513, 1489, 1442, 1409, 1310, 1278, 1219, 1157, 1118, 1092, 1065, 1024, 971, 932, 917, 887, 857, 834, 762, 724, 700, 667, 621, 559, 540, 519, 475 cm⁻¹. The FT-IR spectrum is shown in Figure 3.

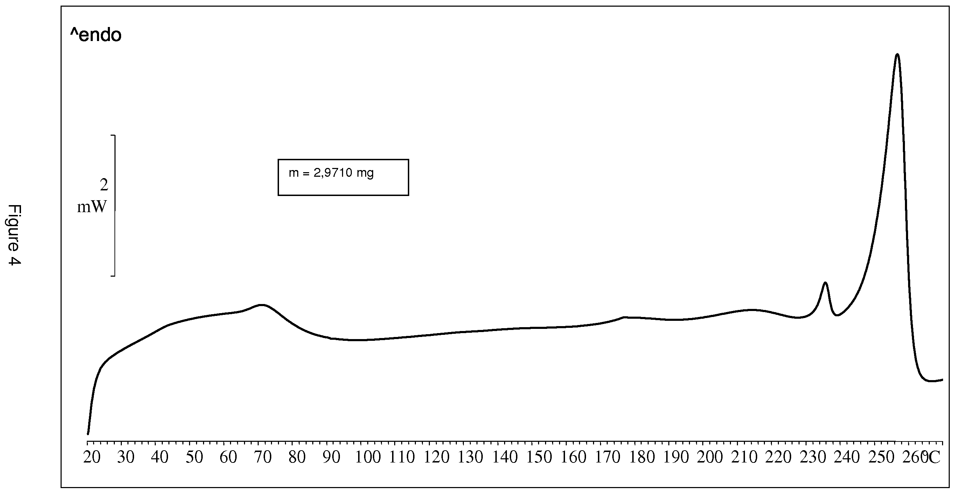

The differential scanning calorimetry (DSC) thermogram of the polymorphic form K shows an endothermic peak (melting with decomposition) at onset temperature 249°C and Tₚₑₐₖ at about 257 °C. Polymorphic form K is further characterized by the DSC thermogram shown in Figure 4.

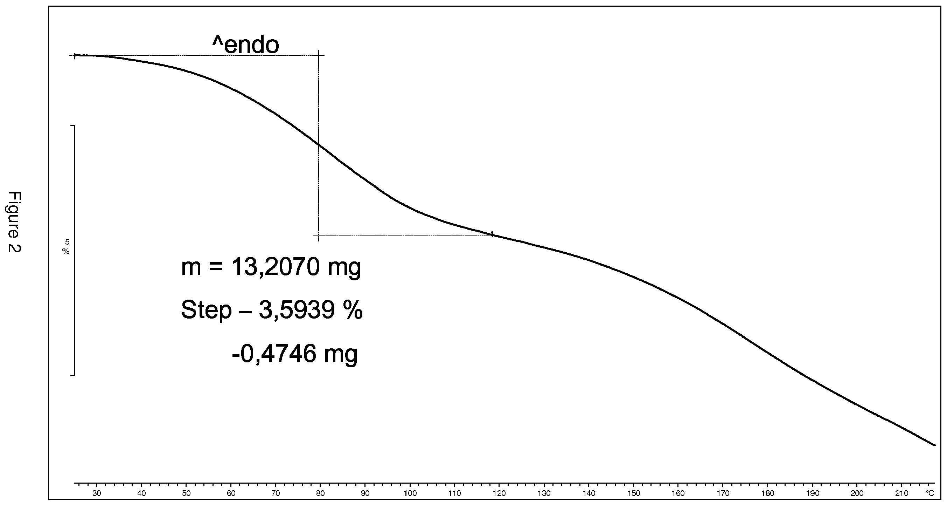

The TG curve is depicted in Figure 2 and shows a weight loss due to water evaporation.

In particular, pitavastatin polymorphic form K is further characterized by a Raman spectrum having bands at 232, 316, 413, 544, 733, 836, 962, 972, 1442, 1648 cm⁻¹.

The pitavastatin calcium in polymorphic forms K according to the present invention is substantially pure.

In the context of the present application, the term "substantially pure" pitavastatin calcium means that the pitavastatin calcium according to the present invention comprises less than 10%, preferably less than 5%, more preferably less than 2%, more preferably less than 1%, and even more preferably less than 0.5% of other crystalline form. Polymorphic purity has been determined by means of X-ray powder diffraction.

The degree of crystallinity may be more than 50%, preferably more than 70% or more than 80%.

In the context of the present application, the term "substantially pure" pitavastatin calcium also means that the pitavastatin calcium according to the present invention comprises less than 3% of other impurities, preferably less than 2%, more preferably less than 1%, and even more preferably less than 0.5% of other impurities.

The present invention also provides a process for the preparing of pitavastatin calcium in polymorphic form K. The process for the preparation of polymorphic form K of pitavastatin calcium according to present invention comprises mixing of an aqueous or substantially aqueous solution of a calcium source with an aqueous or substantially aqueous solution of a water soluble salt of pitavastatin, wherein the solution of the calcium source is added to the solution of the water-soluble salt of pitavastatin at a temperature of between 40°C and 80°C.

Suitable water soluble salts may be metal salts, for example an alkali metal salt, such as sodium, lithium, potassium; or an ammonium salt or a salt with an organic amine, such as primary, secondary amines, preferably sodium salt is used.

Furthermore, a salt with an organic amine can be used for the preparation of the water soluble salt or for preparation of pitavastatin calcium. A suitable organic amine can be selected from the group of methyl amine, ethyl amine, n-propyl amine, isopropyl amine, n-butyl amine, tertiary butyl amine, (+/-)-sec-butyl amine, octyl amine, 2-ethyl hexylamine, benzyl amine, [α]-methyl-benzylamine, (+/-)-phenylethanamine, dibenzylamine, N-methylbenzylamine, N,N-dimethylbenzylamine, N,N-diethyl benzyl amine, N-ethyl-N-methylbenzylamine, tribenzyl amine, cyclopentylamine, cyclohexylamine, cycloheptylamine, N-methylcyclopentylamine, N-ethylcyclohexyl amine, N-ethyl cycloheptylamine, dicyclohexylamine, N,N-dimethylcyclo pentylamine, N,N-dimethyl cyclohexylamine, N,N-diethylcycloheptylamine and the like. Chiral amines can be used in racemate form or in the form of enantiomeres thereof.

Pitavastatin water soluble salt used in the respective processes for the preparation of the polymorphic form K may be prepared according to any method known to the skilled person. Pitavastatin water soluble salt can be prepared according to the methods known in the art, for example according to a method disclosed in EP 304063, EP520406, WO2010089770, WO2007132482, WO 2011/089623.

The solution of the water soluble salt can be prepared by dissolution of a solid form of the salt in water or can be prepared from pitavastatin acid or a suitable derivate thereof, such as an alternative salt form or ester. Water soluble salt, for example sodium salt, can be generated by treatment of an alternative salt, such as methylamine or phenylethanamine salt, with a sodium base, such as sodium hydroxide or sodium carbonate, preferably sodium hydroxide. Similarly, other bases, such as other alkali metal bases could be used to generate solutions of other water-soluble salts.

The calcium source may be selected from the group of calcium chloride, calcium acetate, calcium hydroxide and calcium alkoxide, such as methoxide, ethoxide.

In the context of the present application, the term "substantially aqueous solution" means a solution which may beside water contain a solvent selected from C₁-C₆ alcohols, such as methanol, ethanol, 1-propanol, 2-propanol, 1-butanol, amyl alcohol and 1-hexanol; nitriles such as acetonitrile; ketones such as acetone, methyl ethyl ketone, methyl isobutyl ketone, ethers such as dioxane or THF, methyl tert-butyl ether (MTBE), diisopropyl ether (DIPE); esters such as methyl acetate, ethyl acetate, isopropyl acetate, butyl acetate, or mixtures thereof, wherein the content of the solvent can vary from 0 to 50%, preferably 0-25%, more preferably 0 to 20%, most preferably 0 to15%.

The main process parameter according to the process of the present invention, which is crucial for obtaining the desired polymorphic form, is the temperature at which the two solutions are mixed together and at which the product is precipitated, herein after denoted as "crystallization temperature" and optionally the time period for which the reaction mixture is mixed. In general, the addition of calcium source solution is carried out over a period of time, hereinafter denoted as the "addition time". After the addition of calcium source solution has been completed the mixture is generally stirred for a period of time, hereinafter denoted as the "holding time".

The addition is carried out at a crystallization temperature of between 40°C and 80°C, preferably between 50°C and 70°C, most preferably between 55°C and 65°C. In a further preferred embodiment the addition is carried out at a crystallization temperature of between 50°C and 80°C, more preferably between 55°C and 80°C.

Addition time according to present invention is from 5 to 120 minutes, preferably from 30 to 70 minutes.

The molar ratio between calcium source and water soluble salt of pitavastatin can be in the range of 0.4:1 to 0.6:1, preferably 0.45:1 to 0.55:1, most preferably 0.49: 1 to 0.51:1.

Onset of crystallization in the context of the present invention can be induced either by primary and/or secondary nucleation mechanism.

Primary nucleation can be affected by stirring the crystallization mixture at the specified crystallization temperature as long as the precipitation of crystals is initiated. Stirring of the crystallization batch is performed either by magnetic or mechanical stirrer. In large scale batches preferably a mechanical stirrer is used. The mechanical stirrer can be selected from an impeller, propeller, turbine, paddle or anchor type configuration.

In case of secondary nucleation the crystallization batch is seeded by addition of crystals of pitavastatin calcium in polymorphic form K at the crystallization temperature. The crystallization batch is seeded by addition of crystals of pitavastatin calcium at least once during the holding time when the temperature of the crystallization batch is within the above defined crystallization temperature range in order to avoid excessive supersaturation with respect to pitavastatin calcium and resulting spontaneous crystallization into non-desired polymorphic forms.

The crystallization batch is kept at crystallization temperature for a holding time for crystal formation for at least 1 minute, preferably in the range of 0.5 hour to 30 hours, more preferably 1 to 20 hours. After said holding time at the crystallization temperature, the crystalline particles of pitavastatin calcium are isolated from the mother liquor using conventional separation techniques, e.g. filtration or centrifugation. Alternatively, the crystallization mixture can be cooled prior to isolation to temperatures from 0 to 60°C.

Drying as used herein refers to the removal of solvent residues performed for example by heating and/or vacuum at temperatures between room temperature and 60 °C.

In a further preferred aspect of the invention there is provided a process for preparation of pitavastatin calcium in polymorphic form K, which process comprises addition of substantially aqueous solution of calcium chloride to an aqueous solution of sodium salt of pitavastatin at temperatures of between 40°C and 80 °C, and preferably between 50 °C and 80 °C, more preferably between 55 °C and 80 °C.

In a further preferred aspect of the invention there is provided a process for preparation of pitavastatin calcium in polymorphic form K, which process comprises addition of substantially aqueous solution of calcium chloride to aqueous solution of sodium salt of pitavastatin at temperatures of between 50°C and 70 °C.

In a further preferred aspect of the invention the aqueous solution of pitavastatin sodium salt is generated by treatment of phenylethanamine salt of pitavastatin ((R)-1-phenylethanamine (3R,5S,E)-7-(2-cyclopropyl-4-(4-fluorophenyl)quinolin-3-yl)-3,5-dihydroxyhept-6-enoate) with sodium hydroxide. The phenylethanamine salt of pitavastatin can be prepared according to a process disclosed in EP 520406.

After treatment of (R)-1-phenylethanamine salt with sodium hydroxide phenylethanamine is formed. (R)-1-Phenyletanamine in free base form may be recovered from the reaction mixture by extraction with water immiscible solvent, preferably ketones, cyclic or non-cyclic ethers, esters, halogenated hydrocarbons or mixtures thereof, more preferably ketones such as acetone, methyl ethyl ketone, methyl isobutyl ketone, ethers such as dioxane or THF, methyl tert-butyl ether (MTBE), diisopropyl ether (DIPE); esters such as methyl acetate, ethyl acetate, isopropyl acetate, butyl acetate, or mixtures thereof, most preferably MTBE. The recovered (R)-1-phenylethanamine can be reused in a process of optical resolution of pitavastatin. This recovery process could also be used for other amine salts as described above to obtain the corresponding amine.

The process according to present invention yields homogenous particles in the form of readily filterable individual needles, which are suitable to be incorporated directly into the formulation. However the obtained product may be further subjected to grinding, milling or micronisation if required.

The polymorphic form K of the present invention has a primary particle size as determined by microscopic method (SEM) in particular in the range of 0.1 and 50 µm, preferably in the range of 1 and 20µm, most preferably in the range of 1 and 10 µm. Primary particles are in form of needles or are lath/plate like.

The polymorphic form K has a tendency to form agglomerate particles in the range of 10 and ∼700 µm.

The average particle size of polymorphic K as determined by laser diffraction methods, such as Malvern, may vary from 1 to 100 µm, preferably from 2 to 80 µm, most preferably 3 to 50 µm. Particle size distribution of polymorphic form K according to present invention is in particular such that d(90) is below 250 µm, preferably below 150 µm, most preferably below 100 µm and d(50) is below 100 µm, preferably below 50 µm, most preferably below 30 µm.

It has surprisingly been found that carrying out the precipitation of pitavastatin calcium according to the present invention allows preparation of such salt with a low specific surface area (determined by nitrogen adsorption - BET method), particularly a specific surface area of below 10 m²/g, most preferably 0.5 m²/g to 5 m²/g. Due to the low specific surface area, such salt is less prone to degradation. Furthermore, a low surface area is also indicative of a low porosity. Low porosity has the advantage of reducing the number of pores in which impurities like NaCl can be caught which would be difficult to remove.

The agglomerates of the polymorphic form K of pitavastatin calcium can be reduced and the particle size distribution can be narrowed, which is advantageous for the manufacturing of solid dosage forms, by conventional milling or grinding methods.

For the milling purposes a fluid energy mill, jet mill, ball mill, roller mill or hammer mill is commonly used as milling equipment.

A fluid energy mill, or "micronizer" , is an especially preferred type of mill for its ability to produce particles of small size in a narrow size distribution, i. e., micronized material. As those skilled in the art are aware, micronizers use the kinetic energy of collision between particles suspended in a rapidly moving fluid (typically air or nitrogen) stream to cleave the particles. A jet mill is a preferred fluid energy mill. The suspended particles are injected under pressure into a recirculating particle stream. Smaller particles are carried aloft inside the mill and swept into a vent connected to a particle size classifier such as a cyclone.

The material is fed into the micronization system in a controlled feed rate by means of a screw feeder or a vibratory feeder. The jet mill is operated with controlled air or nitrogen pressures. For the micronizer Jetmill MC-500, the feed rate is 3 - 40 kg/hr, the Feed air/nitrogen pressure is 0.5 - 6 bar and the grinding air is 1 - 6 bar.

Micronization can also be accomplished with an impact pin mill. The starting material may have an average particle size of 20 - 100 microns. The material is fed into the mill system in a controlled feed rate by means of a screw feeder or a vibratory feeder. The mill is operated with controlled speed. For the Alpine UPZ 100, the feed rate is 6 - 40 kg/hr, the mill speed is 7000 - 15000 rpm.

Pitavastatin calcium in polymorphic form K as obtained by the process according to the present invention is surprisingly and unexpectedly stable in terms of chemical and physical stability. We have surprisingly found out that the polymorphic form K according to the present invention does not convert to other polymorphic form when subjected to:
- stress stability conditions at 40°C / 75%RH (relative humidity) for 1 month in an open or closed container
- humidity condition at room temperature of 90% RH (relative humidity) for 3 days
- mechanical stress - compression force of 8 tons/cm²
- grinding (for example in a mortar).

The result of a comparison with polymorphic forms known in the art, such as form A, form A with decreased crystallinity, form E and amorphous form of pitavastatin calcium, which have been tested under the same conditions is shown in Table 1.

**Table 1**

| **Polymorphic form** | **40°C**/**75**%**RH**/ **1m (open)** | **40°C**/**75**%**RH**/ **1m (closed)** | **Compression force of 8 tons**/**cm²** | **25°C/90%RH/ 3days** |
|---|---|---|---|---|
| **Form K** | Form K | Form K | Form K | Form K |
| **amorphous form** | amorphous form | amorphous form | amorphous form | amorphous form |
| **form A** | form A | form A | form A with decreased crystallinity | form A + trace of form E |
| **form E** | form E + trace of form A | form E + trace of form A | form A with decreased crystallinity | form E |
| **form A with decreased crystallinity** | form E | form A with decreased crystallinity | form A with more decreased crystallinity | form E |

As evident from Table 1, the new polymorphic form K proves to be stable when subjected to stress stability conditions or compression. This is not true for other polymorphic forms known in the art, which under the testing conditions undergo changes in the crystal form. On the other hand the amorphous form which is stable in the terms of physical stability is extremely unstable in terms of chemical stability as shown for example in WO 2005/063711, whereas polymorphic form K according to the present invention is stable also in terms of chemical stability.

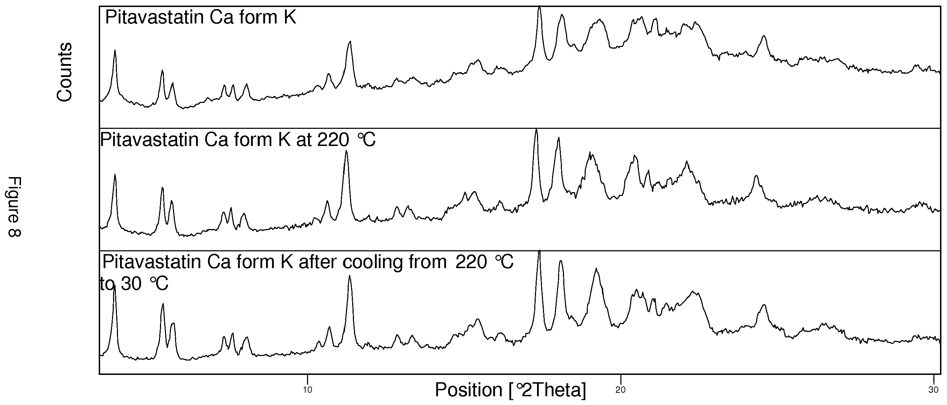

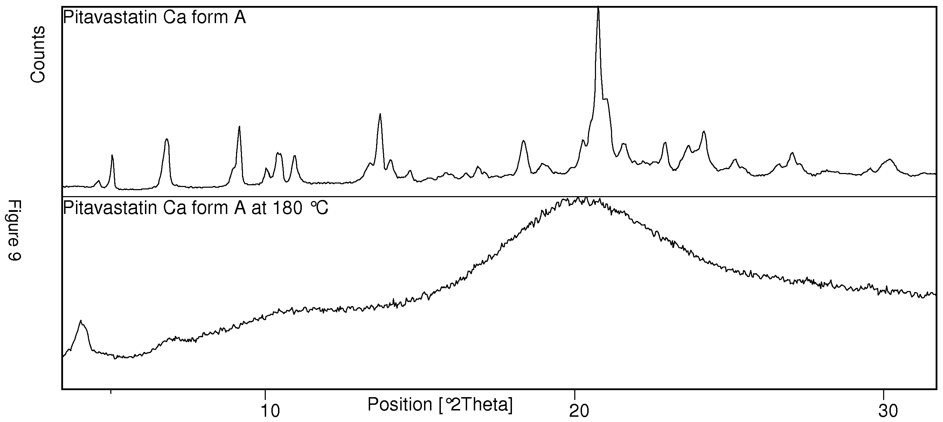

Furthermore, the polymorphic form K according to the present invention shows surprisingly high thermal stability, i.e. stability of a molecule at high temperature. Polymorphic form K is more resistant to decomposition at high temperatures than polymorphic form A according to WO2005/063711, which was known to be the most stable form of pitavastatin calcium. Polymorphic form K decomposes at temperatures above 248°C, whereas polymorphic form A decomposes at temperatures of about 180°C. This is evident from the DSC curve, shown in Figure 4. The powder diffraction pattern of polymorphic form K heated to 220 °C shows only a slight change in comparison to the powder diffraction pattern of initial state (recorded at room temperature). The powder diffraction pattern of a sample which was heated and cooled to room temperature represents form K with better crystallinity (Figure 8). On the other hand, polymorphic form A shows a decrease in crystallinity during the heating process and finally at 180 °C converts to the amorphous state (Figure 9). At 70 °C the powder diffraction pattern of the sample shows form A with decreased crystallinity.

In addition to that, the polymorphic form K according to the present invention is a slightly hygroscopic material in contrast to other known polymorphic materials of pitavastatin calcium which can be referred to as very hygroscopic materials. Hygroscopicity is illustrated by dynamic sorption analysis (DVS) as shown in Table 2.

**Table 2**

| **Polymorphic form** | **Loss on drying at 0 %RH, 25 °C** | **Amount of sorbed water at 90 %RH** |
|---|---|---|
| Amorphous form | -0.8 % | 5.2% |
| Form E | -9.2% | 13.7% |
| Form A with decreased crystallinity | -4.4% | 12.4% |
| Form K | -1.9% | 3.3 % |
| Form A | -8.8% | 13.9% |

The pitavastatin calcium may further be packaged in a package such as a polyethylene bag or hot sealed polyethylene laminated bag in an inert atmosphere, preferably in a hot sealed polyethylene laminated bag in nitrogen atmosphere. Pitavastatin calcium may be stored at temperatures ranging from 0°C to room temperature.

According to another embodiment of the present invention pharmaceutical compositions or formulations comprising polymorphic form K of pitavastatin calcium as an active ingredient are encompassed. Of particular advantage is that the present polymorph form is surprisingly stable when subjected to standard technological processes used in the preparation of pharmaceutical formulations such as granulation, compression or drying.

The crystalline form K of pitavastatin calcium can be formulated into solid dosage forms such as tablets, pellets or capsules. Drug containing solid dosage forms can be manufactured by the state of the art processes such as direct compression, dry granulation, or wet granulation.

In one embodiment of the present invention a pitavastatin calcium containing solid dosage form is produced by direct compression of powder mixture of pitavastatin calcium and at least one further ingredient selected from stabilizers, diluents, binders, disintegrants, glidants and lubricants, where stabilizers used in the weight ratio to drug from 1:10 to 10:1 can be selected from water insoluble alkaline reacting compounds having a pH of the saturated solution of such alkaline reacting substance in purified water of at least 8.0, preferably 8.5 and most preferably 9.0, such as alkaline, earth alkaline or aluminum oxides, hydroxides, carbonates, phosphates, silicates or mixed inorganic salts or water soluble alkaline reacting compounds such as alkali hydroxides, carbonates, di- and tribasic phosphates (e.g. disodium hydrogen phosphate, trisodium phosphate) and organic bases such as meglumine, arginine, tri-ethanolamine or the like. Diluents can be selected from sucrose, lactose, mannitol, dextrose, sorbitol, trehalose, starch and its derivatives, microcrystalline cellulose. Diluents are preferably used in the range of 10 - 95 % (w/w), more preferably in the range of 50 - 90 %. Binders can be selected from water soluble polymers such as soluble cellulose ethers such as hydroxypropylmethyl cellulose (having a viscosity of a 2 % solution in water at 20°C of below 20 cps (determined according to USP method), preferably below 15 cps and most preferably below 10 cps), hydroxypropyl cellulose, which contains not less than 52.0 % and not more than 81.0 % of hydroxypropoxy groups (-OCH2CHOHCH3), hydroxyethyl cellulose, methyl cellulose, povidone, copovidone, polyvinyl alcohol and/or water insoluble polymers such as starch and its derivatives and microcrystalline cellulose. Binders are preferably used in up to 10 % (w/w), more preferably from 1 to 8 % (w/w) and most preferably from 2 to 6 % (w/w). Disintegrants can be selected from crospovidone, sodium carboxymethyl cellulose, calcium carboxymethyl cellulose, sodium starch glycolate, low-substituted hydroxypropyl cellulose which contains not less than 5.0 % and not more than 16.0 % of hydroxypropoxy groups (-OCH2CHOHCH3), polacriline potassium. Low-substituted hydroxypropyl cellulose is preferred. Glidants can be selected from colloidal silicon dioxide (Aerosil), talc, magnesium trisilicate, magnesium silicate, calcium phosphate, powdered cellulose and magnesium oxide. Lubricants can be selected from metal stearates such as magnesium, calcium, zinc or aluminium stearate, sodium starch fumarate, hydrogenated vegetable oils and stearic acid.

In another embodiment of the invention a pitavastatin calcium solid dosage form can be produced via granulation.The granulation process can be performed in the presence of solvent such as wet granulation processes in fluid bed or high shear granulators, where water or organic solvents or the mixtures thereof can be used as granulation liquid or in the absence of solvent such as dry granulation processes like roller compaction or slugging or melt granulation processes are employed. The granulate is formulated by transformation of a powder mixture of pitavastatin calcium according to present invention, stabilizer and at least one further ingredient selected from diluents, binders, disintegrants, glidants, lubricants or surface active substances which can be the same as used in case of direct compression except for binders where beside binders such as water soluble polymers such as soluble cellulose ethers such as hydroxypropyl methylcellulose (having a viscosity of a 2 % solution in water at 20°C of below 20 cps (determined according to USP method), preferably below 15 cps and most preferably below 10 cps), hydroxypropyl cellulose which contains not less than 52.0% and not more than 81.0 % of hydroxypropoxy groups (-OCH2CHOHCH3), hydroxyethyl cellulose, methyl cellulose, povidone, copovidone, polyvinylalcohol and/or water insoluble polymers such as starch and its derivatives, and microcrystalline cellulose, also binders with melting point below 75°C, preferably below 55°C and most preferably between 35 and 45°C such as poloxameres, polyethyleneglycoles with MW below 10,000, partial glycerides, sugar esters or the like are incorporated into granules by the above-mentioned state of the art processes such as dry, wet or melt granulation. Surface active substances can be selected from anionic surfactants such as sodium lauryl sulphate and or non-ionic surfactants such as polysorbates, sugar esters, poloxamers or the like. The obtained granulate is mixed with extragranular excipients selected from stabilizers, diluents, disintegrants, glidants and lubricants and compressed into tablet cores.

Examples of stabilizers are water insoluble alkaline reacting compounds having a pH of the saturated solution of such alkaline reacting substance in purified water of at least 8.0, preferably 8.5 and most preferably 9.0 or water soluble alkaline reacting compounds. Examples of alkaline reacting compounds are alkaline, earth alkaline or aluminum oxides, hydroxides, carbonates, phosphates, silicates, mixed inorganic salts such as magnesium hydroxide carbonate, Magnesium aluminometasilicate, alkali metal hydroxides such as sodium or potassium hydroxide, alkali carbonates, alkali di- or tribasic phosphates such as disodium hydrogen phosphate or trisodium phosphate, organic bases such as meglumine, arginine, triethanolamine, diethanolamine, trihydroxymethylaminomethane, salts of week organic acids such as potassium or sodium citrate, sodium carbonate. Examples of disintegrants are crospovidone, sodium carboxymethyl cellulose, calcium carboxymethyl cellulose, sodium starch glycolate, low-substituted hydroxypropyl cellulose having not less than 5.0% and not more than 16.0% of hydroxypropoxy groups (-OCH2CHOHCH3). Glidants and lubricants can be selected form colloidal silicon dioxide (Aerosil), metal stearates such as magnesium, calcium, zinc or aluminium stearate, sodium starch fumarate, hydrogenated vegetable oils, and stearic acid.

The solid pharmaceutical composition of the present invention can optionally be film-coated with a coating soluble in water. The coating may be applied onto tablet cores by aqueous or organic solvent based film-coating as generally known in the art. According to a particular embodiment, the solid pharmaceutical composition of present invention is coated with a film-coating having a low permeability for gases such as water vapor and/or oxygen. Preferably, the film coating has a water vapor permeability of below 300 g/m²-d (determined according to DIN 53122), preferably below 200 g/m²-d. Typically, the thickness of the film coating is at least 5 µm, particularly at least 10 µm and most preferably at least 15 µm. Film coatings characterized by low permeability for gases such as water vapor and/or oxygen may be based on polymers such as polyvinyl alcohol (e.g. Opadry AMB), low viscosity hypromellose types, hydroxypropylcellulose, sodium carboxymethyl cellulose, aminoalkyl methycrylate copolymers (e.g. Eudragit E PO or Eudragit E 12.5). Further excipients such as plasticizers, anti-tacking agents, pigments and colorants may optionally be incorporated into the coating. Suitable plasticizers include alkyl esters of citric acid (e.g. triethylcitrate), phthalates (e.g. diethylphtalate), dibutylsebacate, triacetin and polyethylene glycol (e.g. PEG 6000, PEG 3000). Suitable anti-tacking agents include glycerol monostearate, stearic acid, talc and/or magnesium stearate. Suitable pigments include metal oxides such as titanium dioxide and/or ferric oxides. Suitable colorants include aluminum lakes (e.g. Indigo Carmine-E132, Allura Red-E129), iron oxides, Curcumin (EIOO) and Carmine (E120). Film coatings can be applied onto tablet cores as a dispersion of polymer and further pharmaceutically acceptable ingredients, such as anti-tacking agents, plasticizers, pigments and/or colorants in a solvent or mixture of solvents selected from water and/or organic solvents such as alcohols (methanol, ethanol, isopropanol or the like), ketones (acetone).

Suitable methods for applying film coatings to tablet cores are generally known in the art, such as coating of tablet cores in perforated coating drums like Manesty, Acela cota, GS or Glatt coating drums.

The invention also relates to a packaged pharmaceutical composition comprising the solid pharmaceutical composition described above, which is present in a low gas permeable primary packaging. The low gas permeable primary packaging may comprise materials such as aluminium or polychloro-3-fluoroethylene homopolymer/PVC laminate. Typically, the thickness of the packaging will be in the range of 10 to 40 µm for Al/Al blisters and 10 to 110 µm for AIlpolychloro-3-fluoroethylene homopolymer/PVC laminate blisters. Optionally, the packaged pharmaceutical composition may further comprise a desiccant. The desiccant may be placed inside the packaging unit together with a dosage form such as a tablet and/or in the closure system and/or can be incorporated into the walls of the primary packaging unit. For example, the pharmaceutical composition can be packaged in containers made of glass or polymers, with or without desiccant.

The premixes as well as the pharmaceutical composition and the final solid oral dosage form may be embedded in a gaseous mixture, wherein the oxygen may be present at a concentration of between approximately 1 % to 16 % (v/v), preferably at a concentration of between approximately 2 to 12 % (v/v), most preferably at a concentration of between approximately 4 to 10 % (v/v). Preferably nitrogen or argon can be used as inert gas atmosphere in the packaging procedure, wherein nitrogen is especially preferred. The pharmaceutical composition of the present invention is packed into primary packaging material under ambient conditions.

In another aspect of the invention pitavastatin calcium in polymorphic form K can be used as to reduce elevated total cholesterol (TC), low-density lipoprotein cholesterol (LDL-C), apolipoprotein B (Apo B), triglycerides (TG), and to increase HDL-C in adult patients with primary hyperlipidemia, including heterozygous familial hypercholesterolaemia, or mixed dyslipidemia.

In a further aspect, the invention also relates to pitavastatin calcium of polymorphic form K for use in combination with ezetimibe, valsartan or amlodipine in the treatment of cardiovascular diseases, hypertension, hypercholesterolaemia and/or obesity in adults.

The following examples illustrate the invention and are not intended to restrict the scope of the invention in any way. All the formulations were prepared using pitavastatin calcium in polymorphic form K according to the present invention.

### Examples

### Methods

**X-ray powder diffraction patterns** were obtained by a Phillips PW3040/60 X'Pert PRO diffractometer using CuKα radiation of 1,541874 Å.

**Images of particles** were taken on a scanning electron microscopeSEM Ultra Plus (Zeiss)

**Particle size** was measured by laser diffraction on a Malvern Mastersizer instrument, equipped with Hydro S dispersion cell using the following settings:
- dispersion medium: Isopar L (RI=1,432)
- sample RI: 1,68
- A: 0,1
- sonication: 20s 50%.

**Raman spectra** were obtained with a BRUKER system Senterra raman microscope. The samples were recorded with a resolution of 4 cm⁻¹ from 3200 cm⁻¹ to 70 cm⁻¹ using a 785 nm laser. The samples are recorded "as is" without any preparation.

**The differential enthalpy analysis (DSC)** was carried out using DSC 822e Mettler Toledo scanning calorimeter. Samples of approx. 3 mg were scanned between 20°C and 270°C at a heating rate of 10°C/min under nitrogen atmosphere.

**The Fourier transform (FTIR) IR spectra** were obtained with a Perkin Elmer system Spectrum GX spectrometer with a resolution of 4 cm⁻¹ from 4000 cm⁻¹ to 400 cm⁻¹ The samples of Form K are prepared in the form of 3% KBr disks. The disks were subjected to a compression of 10 tons for 2 minutes.

**Thermogravimetric (TG)** analysis was performed on a TGA/DSC1 Mettler Toledo analyzer. Samples of approx. 7 - 15 mg were scanned between 20 °C and 220 °C at a heating rate of 10 °C/min under nitrogen atmosphere.

**Specific surface area measurement** was performed with a Micromeritics Tristar 3000. Each sample was degassed at room temperature for 2 hours.

**Dynamic vapor sorption** analysis was performed on a dynamic vapor sorption - DVS Advantage 1 analyzer. Small amounts of samples were exposed to a relative humidity from 0 % to 90 % RH and from 90 % RH back to 0 % in two cycles at 25 °C. Each sorption and desorption have 10 steps. Parameter dmdt = 0,002 %, maximum time is 360 minutes and minimum 10 minutes.

### Chromatographic purity of pitavastatin calcium was determined by HPLC method

The product purity is assessed by high pressure liquid chromatography (HPLC) with a column of the type Ascentis Express C8 150 x 4.6 mm, 2.7 µm; detector: UV245 nm; flow rate: 1.0 ml/min; injection volume: 5 µl; mobile phase: A: 0.02M NH₄COOCH₃ pH=3.8; B: acetonitril; Gradient: 0'=35%B, 10'=40%B, 35'-38'= 85%B, 40'=35%B.

Detailed description of:HPLC method for chromatographic purity
- **Column:**: Ascentis Express C8 150 x 4.6 mm, 2.7 µm,
- **Eluent A:**: 0.02M ammonium acetate pH=3.8
- **Eluent B:**: acetonitrile

Gradient:

| Time (min) | Eluent A (%) | Eluent B (%) |
|---|---|---|
| 0 | 65 | 35 |
| 10 | 60 | 40 |
| 35 | 15 | 85 |
| 38 | 15 | 85 |
| 40 | 65 | 35 |

- **Post time:**: 3 min
- **Flow-rate:**: 1.0 ml/min
- **Detection:**: UV, wavelength 245 nm
- **Injection volume:**: 5 µl
- **Column temperature:**: 40°C
- **Diluent:**: acetonitrile: water (50 : 50, V:V)
- **Method evaluation:**: area per cent

### Preparation of pitavastatin calcium in polymorphic form K

### Example 1

Phenylethanamine salt of pitavastatin ((R)-1-phenylethanamine (3R,5S,E)-7-(2-cyclopropyl-4-(4-fluorophenyl)quinolin-3-yl)-3,5-dihydroxyhept-6-enoate, 6.0 g), 1M NaOH (12.15 ml) and distilled water (100 ml) are charged into a glass reactor, equipped with condenser and mechanical stirrer, under nitrogen atmosphere and protected from light. The temperature of the mixture is maintained at 22°C and it is stirred until a clear solution is formed. After the formation of a clear solution the temperature of the solution is maintained by the means of a thermostat at 60°C. Then a solution of CaCl₂ in water is added gradually in the next 50 minutes (1.47 g CaCl₂x6H₂O in 100 ml of distilled water) and the temperature of the content of the reactor is maintained at 60°C. The formed suspension is then maintained by the means of a thermostat at 60°C and mixed for the next 19 hours. The obtained product is isolated with suction filtration using a Büchner funnel and nitrogen atmosphere. The isolated product is dried in a vacuum dryer under reduced pressure (<100 mbar) and at a temperature of 30°C until the water content is below 6 %.
yield: 3.8 g
water content: 5.7 %

### Example 2

Phenylethanamine salt of pitavastatin ((R)-1-phenylethanamine (3R,5S,E)-7-(2-cyclopropyl-4-(4-fluorophenyl)quinolin-3-yl)-3,5-dihydroxyhept-6-enoate, 50.0 g), 1 M NaOH (104 ml) and distilled water (833 ml) are charged into a glass reactor, equipped with condenser and mechanical stirrer, under nitrogen atmosphere and protected from light. The temperature of the mixture is maintained at 22 °C and it is stirred until a clear solution is formed. After the formation of a clear solution the temperature of the solution is maintained by means of a thermostat at 60 °C. Then a solution of CaCl₂ in water is added gradually in the next 50 minutes (12.25 g CaCl₂x6H₂O in 833 ml of distilled water) and the temperature of the content of the reactor is maintained at 60 °C. The formed suspension is then maintained by means of a thermostat at 60 °C and mixed for the next 20 hours. The obtained product is isolated with suction filtration using a Büchner funnel and nitrogen atmosphere and washed with 5 ml of distilled water. The isolated product is dried in a vacuum dryer under reduced pressure (<100 mbar) and at a temperature of 40°C until the water content is below 4 %.
yield: 26.9 g
water content: 3.6 %
Chromatographic purity of pitavastatin: 99.62 % area HPLC

### Example 3

Phenylethanamine salt of pitavastatin ((R)-1-phenylethanamine (3R,5S,E)-7-(2-cyclopropyl-4-(4-fluorophenyl)quinolin-3-yl)-3,5-dihydroxyhept-6-enoate, 200.0 g), 1 M NaOH (416 ml) and distilled water (1640 ml) are charged into a glass reactor, equipped with condenser and mechanical stirrer, under nitrogen atmosphere and protected from light. The temperature of the mixture is maintained at 22°C and it is stirred until a clear solution is formed. After the formation of a clear solution the mixture was heated to 40°C and MTBE (2000 ml) was added. Immiscible phases were mixed for 15 minutes at the temperature of mixture of 40°C, and left to separate. The phases were separated (organic phase was discarded or used for (R)-1-phenylethanamine recovery) and the water phase was maintained by means of a thermostat at 69°C for further use. After the targeted temperature was reached, 1.3 g of seeds of Form K (pitavastatin Ca) was added. Then a solution of CaCl₂ in water is added gradually in the next 90 minutes (44.0 g CaCl₂x6H₂O in 3280 ml of distilled water, pre-heated to 43 °C) and the temperature of the content of the reactor is maintained at 69°C during the addition. The formed suspension is then maintained by the means of a thermostat at 69°C and mixed for the next 23 hours. The obtained product is isolated with suction filtration using a Büchner funnel and nitrogen atmosphere and washed with 800 ml of distilled water (45°C). The obtained wet product (724 g) is charged into a reactor and distilled water is added (3890 ml, 60°C). The formed suspension was intensively mixed for the next 130 minutes at the temperature of the mixture of 60°C. The obtained product is isolated with suction filtration using a Büchner funnel and nitrogen atmosphere and washed with 260 ml of distilled water (40°C).

The isolated product is dried in a vacuum dryer under reduced pressure (<50 mbar) and at a temperature of 40 to 45°C until a water content of below 1 % is reached.
yield: 105.8 g
water content: 0.5 %
HPLC purity of pitavastatin: 99.78 area %
HPLC assay as is: 98.5 wt. %

### Example 4

Phenylethanamine salt of pitavastatin ((R)-1-phenylethanamine (3R,5S,E)-7-(2-cyclopropyl-4-(4-fluorophenyl)quinolin-3-yl)-3,5-dihydroxyhept-6-enoate, 250.0 g), 1 M NaOH (520 ml) and distilled water (2050 ml) are charged into a glass reactor, equipped with condenser and mechanical stirrer, under nitrogen atmosphere and protected from light. The temperature of the mixture is maintained at 22°C and it is stirred until a clear solution is formed. After the formation of a clear solution the mixture was heated to 40°C and MTBE (2500 ml) was added. Immiscible phases were mixed for 15 minutes at the temperature of the mixture of 40°C, and left to separate. Furthermore, the phases were separated (organic phase was discarded or used for (R)-1-phenylethanamine recovery) and the water phase was maintained by means of a thermostat at 73°C. After the targeted temperature was reached, 2.2 g of seeds of Form K (pitavastatin Ca) was added. Then a solution of CaCl₂ in water is added gradually in the next 105 minutes (55.0 g CaCl₂x6H₂O in 4100 ml of distilled water, pre-heated to 40°C) and the temperature of the content of the reactor is maintained at 73°C during the addition. The formed suspension is then maintained by means of a thermostat at 73°C and mixed for the next 20 hours. The obtained product is isolated with suction filtration using a Büchner funnel and nitrogen atmosphere and washed with 600 ml of distilled water (75°C).

The isolated product is dried in a vacuum dryer under reduced pressure (<50 mbar) and at a temperature of 40 to 50°C until a water content below 2 % is reached.
yield: 171.8 g
water content: 2.0 %
HPLC purity of pitavastatin: 99.88 area %
HPLC assay as is: 98.8 wt. %

### Example 5

Phenylethanamine salt of pitavastatin ((R)-1-phenylethanamine (3R,5S,E)-7-(2-cyclopropyl-4-(4-fluorophenyl)quinolin-3-yl)-3,5-dihydroxyhept-6-enoate, 5.0 g), 1M NaOH (10.4 ml) and distilled water (41 ml) are charged into a glass reactor, equipped with condenser and mechanical stirrer, under nitrogen atmosphere and protected from light. The temperature of the mixture is maintained at 22°C and stirred until a clear solution is formed. After the formation of a clear solution the mixture was heated to 40°C and MTBE (50.0 ml) was added. Immiscible phases were mixed for 15 minutes at the temperature of mixture of 40°C, and left to separate. Furthermore, the phases were separated and the water phase was maintained by means of a thermostat at 73°C and used in the following procedure. After the targeted temperature was reached, 0.05 g of seeds of Form K (pitavastatin Ca) was added. Then a solution of CaCl₂ in water is added gradually in the next 180 minutes (44.0 g CaCl₂x6H₂O in 3280 ml of distilled water, pre-heated to 45°C) and the temperature of the content of the reactor is maintained at 72 - 74°C during the addition. The formed suspension is then maintained by means of a thermostat at 73°C and mixed for the next 18 hours. The obtained product is isolated with suction filtration using a Büchner funnel and nitrogen atmosphere and washed with 20 ml of distilled water (45°C).

The isolated product is dried in a vacuum dryer under reduced pressure (<50 mbar) and at a temperature of 40 to 45°C until a water content below 1 % is reached.
yield: 2.9 g

### Recovery of (R)-1-phenylethanamine:

The separated MTBE phase was distilled using vacuum distillation at a bath temperature of 40°C and a pressure of < 50 mbar until constant mass of evaporated MTBE residue, m = 0.8 g.
water content: 0.6 %
HPLC purity of pitavastatin: 99.75 area %
HPLC assay as is: 98.6 wt. %

### Example 6

Phenylethanamine salt of pitavastatin ((R)-1-phenylethanamine (3R,5S,E)-7-(2-cyclopropyl-4-(4-fluorophenyl)quinolin-3-yl)-3,5-dihydroxyhept-6-enoate, 5.0 g), 1M NaOH (10.4 ml) and distilled water (41 ml) are charged into a glass reactor, equipped with condenser and mechanical stirrer, under nitrogen atmosphere and protected from light. The temperature of the mixture is maintained at 22°C and stirred until a clear solution is formed. After the formation of a clear solution the mixture was heated to 40°C and MTBE (50.0 ml) was added. Immiscible phases were mixed for 15 minutes at the temperature of mixture of 40°C, and left to separate. Furthermore, the phases were separated and the water phase was maintained by means of a thermostat at 73°C and used in the following procedure. After the targeted temperature was reached, a solution of CaCl₂ in water is added gradually in the next 180 minutes (44.0 g CaCl₂x6H₂O in 3280 ml of distilled water, pre-heated to 45°C) and the temperature of the content of the reactor is maintained at 72 - 74°C during the addition. The formed suspension is then maintained by means of a thermostat at 73°C and mixed for the next 18 hours. The obtained product is isolated with suction filtration using a Büchner funnel and nitrogen atmosphere and washed with 20 ml of distilled water (45°C).

The isolated product is dried in a vacuum dryer under reduced pressure (<50 mbar) and at a temperature of 40 to 45°C until water content below 1 % is reached. yield: 3.1 g
water content: 0.6 %
HPLC purity of pitavastatin: 99.78 area %
HPLC assay as is: 98.2 wt. %

### Form A with decreased crystallinity

### Example 7 (Comparative example)

Phenylethanamine salt of pitavastatin ((R)-1-phenylethanamine (3R,5S,E)-7-(2-cyclopropyl-4-(4-fluorophenyl)quinolin-3-yl)-3,5-dihydroxyhept-6-enoate, 200.0 g), NaOH pellets (16.64 g) and distilled water (3736 ml) are charged into a glass reactor, equipped with condenser and mechanical stirrer, under nitrogen atmosphere and protected from light. The temperature of the mixture is maintained at 22°C and it is stirred until a clear solution is formed. After the formation of a clear solution and during following crystallization, the mixture was maintained by means of a thermostat at 22 - 24°C. Then a solution of CaCl₂ in water is added gradually in the next 60 minutes (49.0 g CaCl₂x6H₂O in 3320 ml of distilled water, prepared at RT) and the temperature of the content of the reactor is maintained at 22 - 24 °C during the addition. The formed suspension is then maintained by means of a thermostat at 22 - 24°C °C and mixed for the next 1 hour. The obtained product is isolated with suction filtration using a Büchner funnel and nitrogen atmosphere and washed with 40 ml of distilled water (75°C).

The isolated product is dried in a vacuum dryer under reduced pressure (<50 mbar) and at a temperature of 35°C until a water content below 3 % is reached. yield: 151.5 g
water content: 3.3 %
HPLC purity of pitavastatin: 99.77 area %
HPLC assay as is: 96.3 wt. %
Average particle size: 14 µm, d(90) 36 µm, d(50) 8.5 µm

### Pharmaceutical Composition Examples

Pharmaceutical compositions comprising pitavastatin calcium in polymorphic form K. The pharmaceutical compositions according to present examples can be prepared by direct compression or by dry or wet granulation method.

### Examples F1-F4:

The tablet according to present invention were prepared having the composition mentioned below

| | Ex. F1 | Ex. F2 | Ex. F3 | Ex. F4 |
|---|---|---|---|---|
| Pitavastatin Ca | 1mg | 1mg | 1mg | 1mg |
| Lactose | 101.4 mg | 103.8mg | 93.9 mg | 98.8 mg |
| Hydroxypropyl cellulose | 12.0 mg | 12 mg | 12 mg | 12.0 mg |
| Hydroxypropylmethyl cellulose | 2.0 mg | 2.0 mg | 2.mg | 2.0 mg |
| Magnesium Aluminometasilicate | 2.4 mg | | 9.9 mg | |
| Sodium hidrogencarbonate | | | | 5.0 mg |
| Magnesium Stearate | 1.2 mg | 1.2 mg | 1.2 mg | 1.2 mg |
| Total | 120 mg | 120 mg | 120 mg | 120 mg |

### Example F5:

| | Ex. F5 |
|---|---|
| Pitavastatin Ca | 1.0 mg |
| Calcium carbonate | 2.0 mg |
| Sodium bicarbonate | 2.0 mg |
| Microcrystalline cellulose | 105.8mg |
| Croscarmelose sodium | 3.0 mg |
| polyvinylpirrolidone | 5.0 mg |
| Magnesium stearate | 1.2 mg |

### Example F6:

| | |
|---|---|
| Pitavastatin calcium | 4.18 mg |
| Lactose monohydrate | 100.00 mg |
| Hydroxypropylmethyl cellulose 5cP | 9.60 mg |
| Purified water | q.s. |
| Lactose monohydrate | 124,62 mg |
| Low-substituted hydroxypropyl cellulose LH-21 | 70.00 mg |
| Sodium bicarbonate | 10.00 mg |
| Magnesium stearate | 1.60 mg |
| Ready to make coating suspension Opadry 03H28758 white | q.s |

Pitavastatin calcium (average particle size of 4 µm, with particle size distribution d(10) 0.993 µm, d(50) 2.8 µm, d(90) 7.6 µm) lactose monohydrate and hydroxypropylmethyl cellulose were homogeneously mixed and granulated with water in HSM granulator. The granulate was dried in a fluid bed dryer. Sieved granulate was homogeneously mixed with the rest of lactose, low-substituted hydroxypropyl cellulose, sodium bicarbonate and magnesium stearate to obtain the compression mixture. After compression, the obtained tablets (320 mg/tablet, diameter: 10.0 mm) were film coated with ready to make film coating suspension. Theoretical weight of the film coated tablets was 330 mg.

### Example F7:

| | |
|---|---|
| Pitavastatin calcium | 4.18 mg |
| Lactose monohydrate | 100.00 mg |
| Hydroxypropylmethyl cellulose 5cP | 9.60 mg |
| Magnesium alumino-metasilicate | 26.40 mg |
| Purified water | q.s. |
| Lactose monohydrate | 118.22 mg |
| Low-substituted hydroxypropyl cellulose LH-21 | 60.00 mg |
| Magnesium stearate | 1.60 mg |
| Ready to make coating suspension Opadry | q.s. |
| 03H28758 white | |

Pitavastatin calcium form K (average particle size of 4 µm, with particle size distribution d(10) 0.993 µm, d(50) 2.8 µm, d(90) 7.6 µm)), lactose monohydrate, hydroxypropylmethyl cellulose and magnesium alumino-metasilicate (e.g. Neusilin FL2) were homogeneously mixed and granulated with water in HSM granulator. The granulate was dried in a fluid bed dryer. Sieved granulate was homogeneously mixed with the rest of lactose, low-substituted hydroxypropyl cellulose and magnesium stearate to obtain the compression mixture. After compression, the obtained tablets (320 mg/tablet, diameter: 10.0 mm) were film coated with ready to make film coating suspension. Theoretical weight of the film coated tablets was 330 mg.

### Example F8:

| | |
|---|---|
| Pitavastatin calcium | 4.18 mg |
| Microcrystalline cellulose | 100.00 mg |
| Hydroxypropylmethyl cellulose 5cP | 9.60 mg |
| Purified water | q.s. |
| Microcrystalline cellulose | 154.62 mg |
| Low-substituted hydroxypropyl cellulose LH-21 | 40.00 mg |
| Sodium bicarbonate | 10.00 mg |
| Magnesium stearate | 1.60 mg |
| Ready to make coating suspension Opadry | q.s. |
| 03H28758 white | |

Pitavastatin calcium (average particle size of 14.0 µm), microcrystalline cellulose (e.g. 200) type were homogeneously mixed and granulated with solution of purified water and hydroxypropylmethyl cellulose in a fluid bed granulator. After drying and sieving of the granulate the rest of microcrystalline cellulose (e.g. 102), low-substituted hydroxypropyl cellulose, sodium bicarbonate and magnesium stearate were added to obtain the compression mixture. After compression, the obtained tablets (320 mg/tablet, diameter: 10.0 mm) were film coated with ready to make film coating suspension. Theoretical weight of the film coated tablets was 330 mg.

### Example F9:

| | |
|---|---|
| Pitavastatin calcium | 4.18 mg |
| Microcrystalline cellulose | 100.00 mg |
| Magnesium alumino-metasilicate | 26.40 mg |
| Hydroxypropylmethyl cellulose 5cP | 9.60 mg |
| Purified water | q.s. |
| Microcrystalline cellulose | 138.22 mg |
| Low-substituted hydroxypropyl cellulose LH-21 | 40.00 mg |
| Magnesium stearate | 1.60 mg |
| Ready to make coating suspension Opadry | q.s. |
| 03H28758 white | |

Pitavastatin calcium (average particle size of 14.0 µm), microcrystalline cellulose (e.g. 200) and magnesium alumino-metasilicate (e.g. Neusilin FH2) were homogeneously mixed and granulated with solution of purified water and hydroxypropylmethyl cellulose (e.g. 102) in a fluid bed granulator. After drying and sieving of the granulate the rest of the microcrystalline cellulose, low-substituted hydroxypropyl cellulose and magnesium stearate were added to obtain the compression mixture. After compression, the obtained tablets (320 mg/tablet, diameter: 10.0 mm) were film coated with ready to make film coating suspension. Theoretical weight of the film coated tablets was 330 mg.

### Example F10:

| | |
|---|---|
| Pitavastatin calcium | 4.18 mg |
| Microcrystalline cellulose | 100.00 mg |
| Magnesium alumino-metasilicate | 26.40 mg |
| Hydroxypropylmethyl cellulose 5cP | 9.60 mg |
| Purified water | q.s. |
| Mannitol | 138.22 mg |
| Low-substituted hydroxypropyl cellulose LH-21 | 40.00 mg |
| Magnesium stearate | 1.60 mg |
| Ready to make coating suspension Opadry | q.s. |
| 03H28758 white | |

Pitavastatin calcium (average particle size of 8.5 µm), microcrystalline cellulose (e.g. 200) and magnesium alumino-metasilicate (e.g. Neusilin FH2) were homogeneously mixed and granulated with solution of purified water and hydroxypropylmethyl cellulose in a fluid bed granulator. After drying and sieving of the granulate mannitol, low-substituted hydroxypropyl cellulose and magnesium stearate were added to obtain the compression mixture. After compression, the obtained tablets (320 mg/tablet, diameter: 10.0 mm) were film coated with ready to make film coating suspension. Theoretical weight of the film coated tablets was 330 mg.

### Example F11:

| | |
|---|---|
| Pitavastatin calcium | 4.18 mg |
| Microcrystalline cellulose | 100.00 mg |
| Hydroxypropylmethyl cellulose 5cP | 9.60 mg |
| Purified water | q.s. |
| Sodium bicarbonate | 10.00 mg |
| Mannitol | 154.62 mg |
| Low-substituted hydroxypropyl cellulose LH-21 | 40.00 mg |
| Magnesium stearate | 1.60 mg |
| Ready to make coating suspension Opadry | q.s. |
| 03H28758 white | |

Pitavastatin calcium (average particle size of 8.5 µm) and microcrystalline cellulose (e.g. 200) were homogeneously mixed and granulated with solution of purified water and hydroxypropylmethyl cellulose in a fluid bed granulator. After drying and sieving of the granulate mannitol, low-substituted hydroxypropyl cellulose, sodium hydrogencarbonate and magnesium stearate were added to obtain the compression mixture. After compression, the obtained tablets (320 mg/tablet, diameter: 10.0 mm) were film coated with ready to make film coating suspension. Theoretical weight of the film coated tablets was 330 mg.

### Example F12:

| | |
|---|---|
| Pitavastatin calcium | 4.18 mg |
| Lactose monohydrate | 210.00 mg |
| Starch | 54.42 mg |
| Magnesium alumino-metasilicate | 26.40 mg |
| Hydroxypropylmethyl cellulose 5cP | 9.60 mg |
| Purified water | q.s. |
| Carmellose Calcium | 13.80 mg |
| Magnesium stearate | 1.60 mg |
| Ready to make coating suspension Opadry | q.s. |
| 03H28758 white | |

Pitavastatin calcium (average particle size of 25.0 µm), lactose monohydrate, starch and magnesium alumino-metasilicate (e.g. Neusilin FL2) were homogeneously mixed and granulated with solution of purified water and hydroxypropylmethyl cellulose in a fluid bed granulator. After drying and sieving of the granulate carmellose calcium and magnesium stearate were added to obtain the compression mixture. After compression, the obtained tablets (320 mg/tablet, diameter: 10.0 mm) were film coated with ready to make film coating suspension. Theoretical weight of the film coated tablets was 330 mg.

### Example F13:

| | |
|---|---|
| Pitavastatin calcium | 4.18 mg |
| Lactose monohydrate | 220.00 mg |
| Starch | 60.82 mg |
| Hydroxypropylmethyl cellulose 5cP | 9.60 mg |
| Purified water | q.s. |
| Sodium bicarbonate | 10.00 mg |
| Carmellose Calcium | 13.80 mg |
| Magnesium stearate | 1.60 mg |
| Ready to make coating suspension Opadry | q.s. |
| 03H28758 white | |

Pitavastatin calcium (average particle size of 25.0 µm) lactose monohydrate, starch were homogeneously mixed and granulated with solution of purified water and hydroxypropylmethyl cellulose in a fluid bed granulator. After drying and sieving of the granulate sodium bicarbonate, carmellose calcium and magnesium stearate were added to obtain the compression mixture. After compression, the obtained tablets (320 mg/tablet, diameter: 10.0 mm) were film coated with ready to make film coating suspension. Theoretical weight of the film coated tablets was 330 mg.

### Example F14:

| | |
|---|---|
| Pitavastatin calcium | 4.18 mg |
| Microcrystalline cellulose | 100.00 mg |
| Lactose monohydrate | 187.22 mg |
| Polyvinylpyrrolidone | 16.00 mg |
| Calcium carbonate | 5.00 mg |
| Sodium bicarbonate | 5.00 mg |
| Silica, Colloidal Anhydrous | 1.00 mg |
| Magnesium stearate | 1.60 mg |
| Ready to make coating suspension | q.s. |
| Opadry 03H28758 white | |

Pitavastatin calcium (average particle size of 15.0 µm), microcrystalline cellulose (e.g. 102), lactose monohydrate, polyvinylpyrrolidone, calcium carbonate and sodium bicarbonate were homogeneously mixed. Magnesium stearate and colloidal anhydrous silica were added to obtain the compression mixture. After compression, the obtained tablets (320mg/tablet, diameter: 10.0 mm) were film coated with ready to make film coating suspension. Theoretical weight of the film coated tablets was 330 mg.

### Example F15:

| | |
|---|---|
| Pitavastatin calcium | 4.18 mg |
| Microcrystalline cellulose | 100.00 mg |
| Lactose monohydrate | 170.82 mg |
| Magnesium alumino-metasilicate | 26.40 mg |
| Polyvinylpyrrolidone | 16.00 mg |
| Silica, Colloidal Anhydrous | 1.00 mg |
| Magnesium stearate | 1.60 mg |
| Ready to make coating suspension | q.s. |
| Opadry 03H28758 white | |

Pitavastatin calcium (e.g. average particle size of 15.0 µm), microcrystalline cellulose (e.g. 102), lactose monohydrate, polyvinylpyrrolidone and magnesium alumino-metasilicate (e.g. Neusilin FL2) were homogeneously mixed. Magnesium stearate and colloidal anhydrous silica were added to obtain the compression mixture. After compression, the obtained tablets (320 mg/tablet, diameter: 10.0 mm) were film coated with ready to make film coating suspension. Theoretical weight of the film coated tablets was 330 mg.

## Claims

1. Substantially pure polymorphic form K of pitavastatin calcium, wherein said substantially pure polymorphic form is **characterized in that** is has an X-ray powder diffraction pattern having peaks at 3.8, 11.3, 17.4, 18.1, 24.6 ± 0.2 degrees two-theta, said X-ray powder diffraction pattern being obtained by using CuKα radiation of 1.541874 Å, and that it comprises less than 10% of other crystalline forms of pitavastatin calcium and less than 3% of other impurities and is **characterized by** an X-ray powder diffraction pattern as shown in Figure 1.

2. The polymorphic form K of pitavastatin calcium according to claim 1, which is **characterized in that** it comprises less than 5% of other crystalline forms of pitavastatin calcium.

3. The polymorphic form K of pitavastatin calcium according to claim 2, which is **characterized in that** it comprises less than 1% of other crystalline forms of pitavastatin calcium.

4. The polymorphic form K of pitavastatin calcium according to any one of claims 1 to 3, which is further **characterized by** having peaks at 3.8, 5.3, 11.3, 15.4, 17.4, 18.1, 19.2, 20.6, 22.3, 24.6 ± 0.2 degrees two-theta.

5. The polymorphic form K of pitavastatin calcium according to any preceding claim, **characterized by** an FT-IR spectrum with the following characteristic absorption bands at 3210, 2944, 2909, 1820, 1603, 1559, 1513, 1489, 1442, 1409, 1310, 1278, 1219, 1157, 1118, 1092, 1065, 1024, 971, 932, 917, 887, 857, 834, 762, 724, 700, 667, 621, 559, 540, 519, 475 cm-1, said FT-IR spectrum being obtained from a 3% KBr disc.

6. The polymorphic form K of pitavastatin calcium according to any preceding claim, **characterized by** an FT-IR spectrum as shown in Figure 3, said FT-IR spectrum being obtained from a 3% KBr disc.

7. The polymorphic form K of pitavastatin calcium according to any preceding claim, **characterized by** a DSC thermogram as shown in Figure 4, said DSC thermogram being obtained by using a heating rate of 10°C/min.

8. The polymorphic form K of pitavastatin calcium according to any preceding claim, which has a specific surface area of below 10 m²/g, preferably 0.5 to 5 m²/g, said surface are being measured with a Micromeritics Tristar 3000 with each sample being degassed at room temperature for 2 hours.

9. The polymorphic form K of pitavastatin calcium according to any preceding claim, which is further **characterized by** a Raman spectrum having bands at 232, 316, 413, 544, 733, 836, 962, 972, 1442, 1648 cm⁻¹, said Raman spectrum being obtained by using a 785 nm laser without any sample preparation.

10. A process for preparing the polymorphic form K of pitavastatin calcium according to any one of claims 1 to 9 which comprises mixing of an aqueous or substantially aqueous solution of a calcium source with an aqueous or substantially aqueous solution of a water soluble salt of pitavastatin, wherein the solution of the calcium source is added to the solution of the water-soluble salt of pitavastatin at a temperature of between 40 and 80°C, preferably between 50 and 80°C, more preferably between 55 and 80°C, and wherein said aqueous or substantially aqueous solutions are **characterized in that**, beside water, they contain from 0 to 50% of solvent selected from C₁-C₆ alcohols, nitriles, ketones, ethers, esters, or mixtures thereof.

11. Process according to claim 10, wherein the product is precipitated at a temperature of between 40 and 80°C, preferably between 50 and 80°C, more preferably between 55 and 80°C.

12. Process according to claim 10 or 11, wherein the solution of the calcium source is added over a period of 5 to 120 minutes.

13. Process according to any one of claims 10 to 12, wherein, after the completing of the addition of the solution of the calcium source, the mixture is stirred for at least 1 minute, preferably 0.5 to 30 hours.

14. Process according to any one of claims 10 to 13, which process comprises addition of a substantially aqueous solution of calcium chloride to an aqueous solution of the sodium salt of pitavastatin at a temperature of between 40 and 80°C, preferably between 50 and 80°C and more preferably between 55 and 80°C.

15. Pharmaceutical composition comprising the polymorphic form K of pitavastatin calcium according to any one of claims 1 to 9 and pharmaceutically acceptable excipients.

16. The polymorphic form K according to any of claims 1 to 9 for use in the reduction of elevated total cholesterol (TC), low-density lipoprotein cholesterol (LDL-C), apolipoprotein B (Apo B), triglycerides (TG), and for increasing HDL-C in adult patients with primary hyperlipidemia, including heterozygous familial hypercholesterolaemia, or mixed dyslipidemia.

17. The polymorphic form K according to any one of claims 1 to 9 for use in combination with ezetimibe, valsartan or amlodipine in the treatment of cardiovascular diseases, hypertension, hypercholesterolaemia and/or obesity in adults.

## Patentansprüche

1. Im Wesentlichen reine polymorphe Form K von Pitavastatincalcium, wobei die im Wesentlichen reine polymorphe Form **dadurch gekennzeichnet ist, dass** sie ein Pulverröntgenbeugungsmuster mit Peaks bei 3,8, 11,3, 17,4, 18,1, 24,6 ± 0,2 Grad 2-Theta hat, wobei das Pulverröntgenbeugungsmuster unter Verwendung von CuKα-Strahlung von 1,541874 Å erhalten worden ist, und dass sie weniger als 10 % andere kristalline Formen von Pitavastatincalcium und weniger als 3 % andere Verunreinigungen aufweist und durch ein Pulverröntgenbeugungsmuster wie in Figur 1 gezeigt gekennzeichnet ist.

2. Polymorphe Form K von Pitavastatincalcium nach Anspruch 1, die **dadurch gekennzeichnet ist, dass** sie weniger als 5 % andere kristalline Formen von Pitavastatincalcium aufweist.

3. Polymorphe Form K von Pitavastatincalcium nach Anspruch 2, die **dadurch gekennzeichnet ist, dass** sie weniger als 1 % andere kristalline Formen von Pitavastatincalcium aufweist.

4. Polymorphe Form K von Pitavastatincalcium nach einem der Ansprüche 1 bis 3, die außerdem **dadurch gekennzeichnet ist, dass** sie Peaks bei 3,8, 5,3, 11,3, 15,4, 17,4, 18,1, 19,2, 20,6, 22,3, 24,6 ± 0,2 Grad 2-Theta hat.

5. Polymorphe Form K von Pitavastatincalcium nach einem der vorangehenden Ansprüche, **gekennzeichnet durch** ein FT-IR-Spektrum mit den folgenden charakteristischen Absorptionsbanden bei 3210, 2944, 2909, 1820, 1603, 1559, 1513, 1489, 1442, 1409, 1310, 1278, 1219, 1157, 1118, 1092, 1065, 1024, 971, 932, 917, 887, 857, 834, 762, 724, 700, 667, 621, 559, 540, 519, 475 cm⁻¹, wobei das FT-IR-Spektrum unter Verwendung einer 3 % KBr-Scheibe erhalten worden ist.

6. Polymorphe Form K von Pitavastatincalcium nach einem der vorangehenden Ansprüche, **gekennzeichnet durch** ein FT-IR-Spektrum wie in Figur 3, wobei das FT-IR-Spektrum unter Verwendung einer 3 % KBr-Scheibe erhalten worden ist.

7. Polymorphe Form K von Pitavastatincalcium nach einem der vorangehenden Ansprüche, **gekennzeichnet durch** ein DSC-Thermogramm wie in Figur 4 gezeigt, wobei das DSC-Thermogramm unter Verwendung einer Heizrate von 10°C/min erhalten worden ist.

8. Polymorphe Form K von Pitavastatincalcium nach einem der vorangehenden Ansprüche, die eine spezifische Oberfläche von weniger als 10 m²/g, vorzugsweise 0,5 bis 5 m²/g, aufweist, wobei die Oberfläche mit einem Micromeritics Tristar 3000 gemessen und jede Probe bei Raumtemperatur für zwei Stunden entgast wurde.

9. Polymorphe Form K von Pitavastatincalcium nach einem der vorangehenden Ansprüche, die außerdem durch ein Raman-Spektrum mit Banden bei 232, 316, 413, 544, 733, 836, 962, 972, 1442, 1648 cm⁻¹ gekennzeichnet ist, wobei das Raman-Spektrum unter Verwendung eines 785-nm-Lasers ohne irgendeine Probenvorbereitung erhalten worden ist.

10. Verfahren zur Herstellung der polymorphen Form K von Pitavastatincalcium nach einem der Ansprüche 1 bis 9, bei dem eine wässrige oder im Wesentlichen wässrige Lösung einer Calciumquelle mit einer wässrigen oder im Wesentlichen wässrigen Lösung eines wasserlöslichen Salzes von Pitavastatin gemischt wird, wobei die Lösung der Calciumquelle zu der Lösung des wasserlöslichen Salzes von Pitavastatin bei einer Temperatur zwischen 40 und 80°C, vorzugsweise zwischen 50 und 80°C, besonders bevorzugt zwischen 55 und 80°C, zugegeben wird und wobei die wässrigen oder im Wesentlichen wässrigen Lösungen **dadurch gekennzeichnet sind, dass** sie neben Wasser 0 bis 50 % Lösungsmittel ausgewählt aus C₁-C₆-Alkoholen, Nitrilen, Ketonen, Ethern, Estern oder Mischungen davon enthalten.

11. Verfahren nach Anspruch 10, bei dem das Produkt bei einer Temperatur zwischen 40 und 80°C, vorzugsweise zwischen 50 und 80°C, besonders bevorzugt 55 und 80°C, ausgefällt wird.

12. Verfahren nach Anspruch 10 oder 11, bei dem die Lösung der Calciumquelle über einen Zeitraum von 5 bis 120 Minuten zugegeben wird.

13. Verfahren nach einem der Ansprüche 10 bis 12, bei dem nach Abschluss der Zugabe der Lösung der Calciumquelle die Mischung für mindestens 1 Minute, vorzugsweise 0,5 bis 30 Stunden, gerührt wird.

14. Verfahren nach einem der Ansprüche 10 bis 13, bei dem eine im Wesentlichen wässrige Lösung von Calicumchlorid zu einer wässrigen Lösung des Natriumsalzes von Pitavastatin bei einer Temperatur zwischen 40 und 80°C, vorzugsweise zwischen 50 und 80°C und besonders bevorzugt zwischen 55 und 80°C zugegeben wird.

15. Pharmazeutische Zusammensetzung, die die polymorphe Form K von Pitavastatincalcium nach einem der Ansprüche 1 bis 9 und pharmazeutisch annehmbare Hilfsstoffe enthält.

16. Polymorphe Form K nach einem der Ansprüche 1 bis 9 zur Verwendung bei der Verringerung von erhöhtem Gesamtcholesterin (TC), niedrigdichtem Lipoprotein-Cholesterin (LDL-C), Apolipoprotein B (Apo B), Triglyceriden (TG) und zur Steigerung von HDL-C bei erwachsenen Patienten mit primärer Hyperlipidämie, einschließlich heterzygoter Hypercholesterinämie, oder gemischter Dyslipidämie.

17. Polymorphe Form K nach einem der Ansprüche 1 bis 9 zur Verwendung in Kombination mit Ezetimib, Valsartan oder Amlodipin bei der Behandlung von kardiovaskulären Erkrankungen, Bluthochdruck, Hypercholesterinämie und/oder Fettleibigkeit bei Erwachsenen.

## Revendications

1. Forme polymorphe K sensiblement pure du sel de calcium de pitavastatine, laquelle forme polymorphe sensiblement pure est **caractérisée en ce qu'**elle donne, à l'état de poudre, un diagramme de diffraction des rayons X présentant des pics pour les valeurs 3,8°, 11,3°, 17,4°, 18,1° et 24,6°, à ± 0,2° près, de l'angle 2θ, ce diagramme de diffraction des rayons X à l'état de poudre étant obtenu avec la raie Kα du cuivre de longueur d'onde 1,541874 Å, et **en ce qu'**elle contient moins de 10 % d'autres formes cristallines du sel de calcium de pitavastatine et moins de 3 % d'autres impuretés et est **caractérisée par** un diagramme de diffraction des rayons X à l'état de poudre tel que présenté sur la figure 1.

2. Forme polymorphe K du sel de calcium de pitavastatine, conforme à la revendication 1, **caractérisée en ce qu'**elle contient moins de 5 % d'autres formes cristallines du sel de calcium de pitavastatine.

3. Forme polymorphe K du sel de calcium de pitavastatine, conforme à la revendication 2, **caractérisée en ce qu'**elle contient moins de 1 % d'autres formes cristallines du sel de calcium de pitavastatine.

4. Forme polymorphe K du sel de calcium de pitavastatine, conforme à l'une des revendications 1 à 3, **caractérisée en ce qu'**elle donne des pics correspondant aux valeurs 3,8°, 5,3°, 11,3°, 15,4°, 17,4°, 18,1°, 19,2°, 20,6°, 22,3° et 24,6°, à ± 0,2° près, de l'angle 2θ.

5. Forme polymorphe K du sel de calcium de pitavastatine, conforme à l'une des revendications précédentes, **caractérisée par** un spectre FT-IR présentant des bandes d'absorption situées aux longueurs d'ondes caractéristiques suivantes, données en cm⁻¹ : 3210, 2944, 2909, 1820, 1603, 1559, 1513, 1489, 1442, 1409, 1310, 1278, 1219, 1157, 1118, 1092, 1065, 1024, 971, 932, 917, 887, 857, 834, 762, 724, 700, 667, 621, 559, 540, 519 et 475, ce spectre FT-IR étant obtenu sur disque de KBr à 3 %.

6. Forme polymorphe K du sel de calcium de pitavastatine, conforme à l'une des revendications précédentes, **caractérisée par** un spectre FT-IR tel que présenté sur la figure 3, ce spectre FT-IR étant obtenu sur disque de KBr à 3 %.

7. Forme polymorphe K du sel de calcium de pitavastatine, conforme à l'une des revendications précédentes, **caractérisée par** un thermogramme d'AED (analyse enthalpique différentielle) tel que présenté sur la figure 4, ce thermogramme d'AED étant obtenu pour une vitesse de chauffage de 10 °C/min.

8. Forme polymorphe K du sel de calcium de pitavastatine, conforme à l'une des revendications précédentes, qui présente une aire sépcifique inférieure à 10 m²/g et de préférence de 0,5 à 5 m²/g, cette aire étant mesurée, à l'aide d'un appareil Micromeritics Tristar 3000, sur des échantillons ayant chacun subi 2 heures de dégazage à température ambiante.

9. Forme polymorphe K du sel de calcium de pitavastatine, conforme à l'une des revendications précédentes, **caractérisée en outre par** un spectre Raman présentant des bandes situées aux longueurs d'ondes suivantes, données en cm⁻¹ 232, 316, 413, 544, 733, 836, 962, 972, 1442 et 1648, ce spectre Raman étant obtenu à l'aide d'un laser à 785 nm, sans aucune préparation de l'échantillon.

10. Procédé de préparation de la forme polymorphe K du sel de calcium de pitavastatine, conforme à l'une des revendications 1 à 9, qui comporte le fait de mélanger une solution aqueuse ou sensiblement aqueuse d'une source de calcium avec une solution aqueuse ou sensiblement aqueuse d'un sel hydrosoluble de pitavastatine, dans laquelle opération l'on ajoute la solution de la source de calcium à la solution de sel hydrosoluble de pitavastatine à une température de 40 à 80 °C, de préférence de 50 à 80 °C et mieux encore de 55 à 80 °C, et dans lequel procédé lesdites solutions aqueuses ou sensiblement aqueuses se caractérisent en ce qu'elles contiennent, outre de l'eau, de 0 à 50 % d'un solvant choisi parmi les alcools en C₁-C₆, nitriles, cétones, éthers et esters ou les mélanges de tels composés.

11. Procédé conforme à la revendication 10, dans lequel on fait précipiter le produit à une température de 40 à 80 °C, de préférence de 50 à 80 °C et mieux encore de 55 à 80 °C.

12. Procédé conforme à la revendication 10 ou 11, dans lequel on effectue l'addition de la solution de la source de calcium en un laps de temps de 5 à 120 minutes.

13. Procédé conforme à l'une des revendications 10 à 12, dans lequel, après avoir achevé d'ajouter la solution de la source de calcium, on agite le mélange durant au moins 1 minute, et de préférence, durant de 0,5 à 30 heures.

14. Procédé conforme à l'une des revendications 10 à 13, lequel procédé comporte le fait d'ajouter une solution sensiblement aqueuse de chlorure de calcium à une solution aqueuse de sel de sodium de pitavastatine, à une température de 40 à 80 °C, de préférence de 50 à 80 °C et mieux encore de 55 à 80 °C.

15. Composition pharmaceutique comprenant de la forme polymorphe K du sel de calcium de pitavastatine, conforme à l'une des revendications 1 à 9, et des excipients pharmacologiquement admissibles.

16. Forme polymorphe K, conforme à l'une des revendications 1 à 9, pour utilisation en vue de faire baisser des taux élevés de cholestérol total (TC), de cholestérol-lipoprotéine basse densité (LDL-C), d'apolipoprotéine B (Apo B) ou de triglycérides (TG), ou de faire augmenter le taux d'HDL-C chez des patients adultes présentant une hyperlipidémie primaire, y compris une hypercholestérolémie familiale hétérozygote, ou une dyslipidémie mixte.

17. Forme polymorphe K, conforme à l'une des revendications 1 à 9, pour utilisation en combinaison avec de l'ézétimibe, du valsartan ou de l'amlodipine dans le traitement de maladies cardio-vasculaires, d'une hypertension, d'une hypercholestérolémie et/ou d'une obésité chez des adultes.
